# EUROPEAN PATENT SPECIFICATION

(11) **EP 3 636 163 B1**
(45) Date of publication and mention of the grant of the patent: **25.10.2023**
(21) Application number: 18794619.9
(22) Date of filing: 03.05.2018
(51) Int. Cl.: A61B 17/00, A61B 17/29, A61B 17/30

(54) **MICROSURGICAL INSTRUMENT CAPABLE OF JOINT MOTION AND ROTATIONAL MOTION**
MIKROCHIRURGISCHES INSTRUMENT FÜR GELENKBEWEGUNG UND DREHBEWEGUNG
INSTRUMENT MICROCHIRURGICAL CAPABLE DE MOUVEMENT D'ARTICULATION ET DE MOUVEMENT DE ROTATION

(30) Priority: 04.05.2017 KR 20170056978
(43) Date of publication of application: 15.04.2020
(73) Proprietor: Ajou University Industry-Academic Cooperation Foundation and Technology, Suwon-si, Gyeonggi-do 16499 (KR)
(72) Inventor: ROH, Tae Hoon, Seongnam-Si Gyeonggi-do 13436 (KR)
(74) Representative: Taor, Simon Edward William
(86) International application number: PCT/KR2018/005129
(87) International publication number: WO 2018/203675

(56) References cited:
- DE-U1-202012 102 477
- JP-A- 2014 530 651
- KR-A- 20120 111 346
- KR-B1- 101 586 177
- US-A- 5 976 164
- US-A1- 2004 267 164
- US-A1- 2012 109 186
- US-A1- 2012 245 567
- US-A1- 2014 012 085
- US-A1- 2015 164 524

## Description

### BACKGROUND OF THE INVENTION

### Field of the invention

The present disclosure relates to a fine surgical instrument capable of performing a joint movement and a rotational movement, and more particularly, a fine surgical instrument capable of operating an affected part in a narrow area such as an endoscopic operation by finely adjusting a direction and an angle and performing a joint movement and rotational movement.

### Related Art

In general, in the related art, when cranial surgery, laparotomy, and incision surgery are performed so as to treat a patient, an incision site is large and a lot of blood is lost during surgery. Accordingly, a recovery of the patient after the surgery is slow, a large scar remains after the surgery, which interferes a life of the patient after the surgery. In order to overcome a disadvantage of the laparotomy surgery, in recent years, a new surgical technique such as minimal invasive surgery (MIS) or natural Orifice transluminal endoscopic surgery (NOTES) has been developed.

The minimal invasive surgery is a surgical technique which incises and treats a minimal portion of a body of a patient using a long and thin surgical tool specifically designed to minimize the incision site required for the surgery, and the natural Orifice transluminal endoscopic surgery is a surgical technique which inserts the surgical tool into a hole (esophagus, anus, vagina, or the like) which naturally presents in a body of a human without incising the body of the patient in order to transfer the surgical tool to a treatment site inside the body of the patient, transfer the surgical tool to the treatment site in the body, and treats the treatment site.

Compared to the laparotomy, in the minimal invasive surgery and the natural Orifice transluminal endoscopic surgery, the incision site required for the treatment is small, an amount of bleeding during the treatment is very small, a recovery period of the patient after the surgery is fast, and the scar which externally appears is small. Accordingly, in recent years, the number of treatments increases significantly.

When the minimal invasive surgery and the natural Orifice transluminal endoscopic surgery are performed, a specially designed endoscope device is used to secure a field of view of a surgery site through a minimal body incision. That is, the endoscope device is a medical imaging device for the minimal invasive surgery in which the field of view inside the body of the patient is not directly secured. A doctor and a nurse who perform the minimal invasive surgery perform the surgery while viewing an image of the treatment sites obtained through the endoscope device.

While the minimal invasive surgery and the natural Orifice transluminal endoscopic surgery are performed, a position and direction of an endoscope should be continuously changed to illuminate a status of the treatment site and a movement of the surgical tool in the body of the patient changed momentarily, as broadly and accurately as possible.

Moreover, in recent years, the endoscope surgery on a base of a brain has been actively performed in a field of neurosurgery. Although not necessarily in the field of the neurosurgery, the minimal invasive surgery using the endoscopes is developed, and thus, a development of an instrument according to the development of the minimal invasive surgery is urgently required.

In a case where there is an enough space in an abdominal cavity and the instrument enters through various ports like general surgery, there is no difficulty in performing the surgery even with an instrument having a straight structure. However, in the surgery on the base of the brain through a nose, a size of an entrance is 2 cm or less, and a depth thereof is about 10 cm or more. Because the base of the brain has a very deep and narrow structure, even when the base of the brain can be viewed if the endoscope enters, in most cases, manipulation is not performed using the straight instrument.

Therefore, in the surgery in the field of the neurosurgery, a surgical device which can continuously change a direction even in a small space is urgently required.

US2014012085A1 discloses a torque-transmitting, variably-flexible device, comprising a hollow body having a proximal end and a distal end, a torque-transmitting element operable to transmit torque from the proximal end towards the distal end of the hollow body, and a steering element operable to steer the distal end of the hollow body.

US2012245567A1 discloses a surgical instrument that includes an instrument shaft having proximal and distal ends, a tool disposed from the distal end of the instrument shaft, a control handle disposed from the proximal end of the instrument shaft, a distal motion member for coupling the distal end of the instrument shaft to the tool, a proximal motion member for coupling the proximal end of the instrument shaft to the handle, actuation means extending between the distal and proximal motion members for coupling motion of the proximal motion member to the distal motion member for controlling the positioning of the tool and a locking mechanism for fixing the position of the tool at a selected position and having locked and unlocked states.

US2015164524A1 discloses an articulation mechanism for a surgical instrument, including an articulation assembly, a plurality of cables, and a trigger. The cables are coupled to the articulation assembly at a proximal end thereof and extend distally therefrom. The cables are configured to engage an end effector assembly of the surgical instrument at a distal end thereof. The trigger is coupled to the articulation assembly and is selectively moveable from a shipping position to a use position. In the shipping position, the cables are substantially un-tensioned. In the use position, the cables are disposed in an initial tensioned position. In the use position, the trigger is moveable between an unlocked position and a locked position. In the unlocked position, the cables are selectively tensionable to articulate the end effector assembly. In the locked position, the tensions on the cables are maintained to lock the end effector assembly in position.

US2012109186A1 discloses an articulating laparoscopic instrument including a handle body, a shaft, an end effector, an operation mechanism, and an articulation mechanism. The end effector is connected to a shaft end. The operation mechanism includes a rod and an actuator. The rod is coupled to the end effector. Movement of the actuator relative to the handle body transfers a force onto the rod in a longitudinal direction to operate the end effector. The articulation mechanism includes a deflection assembly, first and second collar assemblies, first and second cables, a linkage, and an articulation actuator. The first and second collar assemblies are slidably disposed over the rod. The cables extend between the collar assemblies and the deflection assembly. Movement of the articulation actuator drives the first and second collar assemblies in opposite directions to cause a longitudinal deflection in the deflection assembly.

DE202012102477U discloses an electrosurgical instrument having an energizable bipolar that is provided with two grumbling portions. The grumbling portions are mechanically adjusted through an axially adjustable pull bar. The pull bar is received in an axially extending sleeve and is detachably connected in a slide portion of a gripping portion. The steering wheels are formed with slide portion. The slide portion is connected to grip shells. The grip shells are arranged on the left and right side of the pull bar and are moved laterally to the adjustment direction.

### SUMMARY OF THE INVENTION

The present disclosure is made to solve the above-described problems of the related art, and provides a fine surgical instrument capable of performing a joint movement and a rotational movement, in which a distal end of the instrument is link-rotated when surgery such as endoscope surgery is performed in a narrow space and the instrument is freely manipulated without colliding with an endoscope.

The present invention is directed to a fine surgical instrument as set out in claim 1. Optional features are set out in the dependent claims.

### BRIEF DESCRIPTION OF THE DRAWINGS

FIG. 1 is a perspective view schematically showing an appearance of a fine surgical instrument capable of performing a joint movement and a rotational movement according to an embodiment of the present disclosure.
FIG. 2 is a cross-sectional view of a rod portion showing a wire according to the embodiment of the present disclosure.
FIG. 3 is a cross-sectional view schematically showing a cross section of a switch.
FIG. 4 is a conceptual view showing a shape in which a joint portion is link-rotated in one direction.
FIG. 5 is a cross-sectional view schematically showing a cross section of a handle portion.
FIG. 6 is a perspective view schematically showing an appearance of a fine surgical instrument capable of performing a joint movement and a rotational movement according to another embodiment of the present disclosure.
FIG. 7 is a conceptual view showing a shape in which a pair of joint portions is link-rotated in the same direction.
FIG. 8 is a conceptual view showing a shape in which the pair of joint portions is link-rotated in different directions.
FIG. 9 is a perspective view schematically showing a shape in which a plurality of link joints and a plurality of wires are coupled.
FIG. 10 is a perspective view schematically showing an appearance of a fine surgical instrument capable of performing a joint movement and a rotational movement according to still another embodiment of the present disclosure.
FIG. 11 is a conceptual view showing a shape in which a pair of joint portions of FIG. 10 is link-rotated in the same direction.
FIG. 12 is a conceptual view showing a shape in which the pair of joint portions of FIG. 10 is link-rotated in different directions.

### DESCRIPTION OF EXEMPLARY EMBODIMENTS

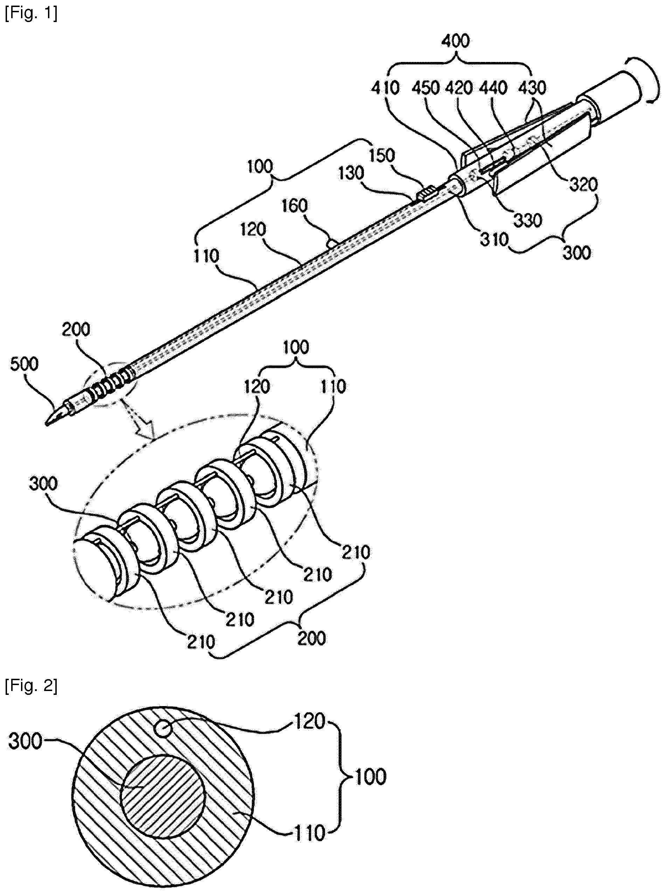

FIG. 1 Hereinafter, preferred embodiments of the present disclosure will be described in detail with reference to the accompanying drawings. First, in adding reference numerals to the components of each drawing, it should be noted that the same reference numerals are used as much as possible even if shown on different drawings. Moreover, in descriptions of the present disclosure, if it is determined that detailed descriptions of known related configurations or functions may obscure a subject matter of the present disclosure, detailed descriptions thereof will be omitted.

Throughout the specification, when a portion is "connected" to another portion, it includes not only "direct connection" but also "indirect connection" via another member therebetween. In addition, when a portion is said to "include" a certain component, this means that unless otherwise stated, it may further include other components rather than excluding the other components.

Moreover, a fine surgical instrument capable of performing a joint movement and a rotational movement according to an embodiment of the present disclosure may be applied to laparoscopic surgery using an endoscope and minimal invasive surgery using an endoscope. Moreover, a fine surgical instrument capable of performing a joint movement and a rotational movement according to an embodiment of the present disclosure may be applied to surgery on a base of a brain through a nasal cavity. In addition, a fine surgical instrument capable of performing a joint movement and a rotational movement according to an embodiment of the present disclosure may be applied to other non-claimed instruments such as a catheter and a medical instrument including diagnostic and therapeutic instruments and tools.

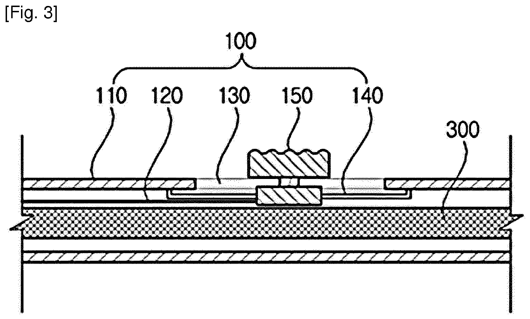

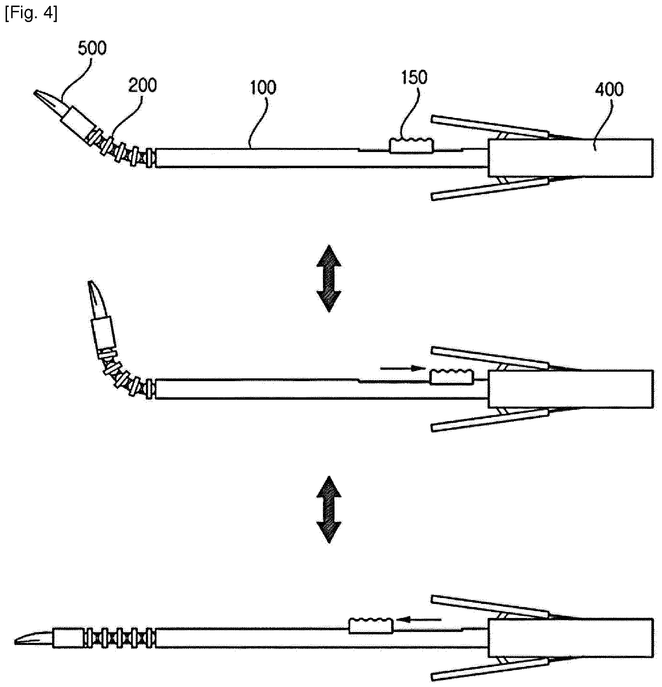

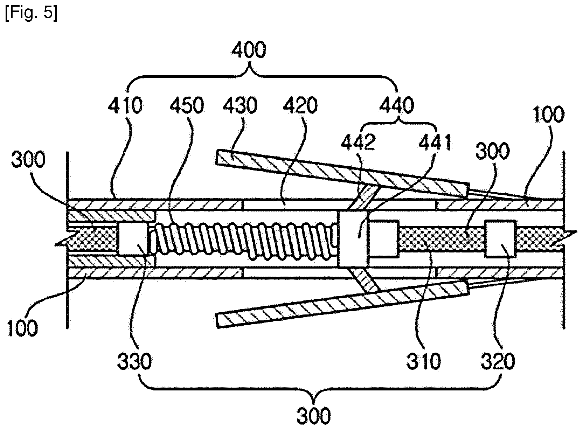

FIG. 1 is a perspective view schematically showing an appearance of a fine surgical instrument capable of performing a joint movement and a rotational movement according to an embodiment of the present disclosure, FIG. 2 is a cross-sectional view of a rod portion showing a wire according to the embodiment of the present disclosure, FIG. 3 is a cross-sectional view schematically showing a cross section of a switch, FIG. 4 is a conceptual view showing a shape in which a joint portion is link-rotated in one direction, and FIG. 5 is a cross-sectional view schematically showing a cross section of a handle portion.

As shown in FIGS. 1 to 5, the fine surgical instrument capable of performing a joint movement and a rotational movement according to an embodiment of the present disclosure includes a rod portion 100, a joint portion 200, a shaft 300, a handle portion 400, and a distal end portion 500.

More specifically, the fine surgical instrument capable of performing a joint movement and a rotational movement according to the embodiment of the present disclosure includes the rod portion 100 which is formed in a longitudinal direction to enter a surgery site in a narrow space, the joint portion 200 which is formed on one side of the rod portion and rotates in one direction, the shaft 300 which is inserted into the rod portion, the handle portion 400 which is coupled with the other side of the rod portion, and the distal end portion 500 which is coupled with one end of the shaft and is connected to the handle portion, in which the distal end portion 500 is operated according to a manipulation of the handle portion 400.

Hereinafter, the fine surgical instrument capable of performing a joint movement and a rotational movement according to the embodiment of the present disclosure will be described in more detail with reference to FIGS. 1 and 2.

The rod portion 100 may be formed in a tubular shape having a small diameter such that a surgery instrument enters the rod portion 100 without colliding with the endoscope. To this end, the rod portion 100 may include a body 110 and a wire 120. In addition, it is preferable that the rod portion 100 has a small diameter for fine surgery. Although the diameter of the rod portion 100 is represented in large in the drawings, it is preferable that the rod portion 100 has the diameter of 2 mm to 3 mm.

The body 110 may be formed in a tubular shape extending in the longitudinal direction. That is, the body 110 may be formed through the center thereof, and the shaft 300 described later may be inserted into the body 110. Referring to FIG. 2, the body 110 is formed of a cross section having an edge at a predetermined thickness. In addition, an insertion hole is formed in a circumferential direction of the cross section. Here, the insertion hole may be a space in which the wire 120 is inserted and disposed.

The wire 120 may be formed along the longitudinal direction of the body 110 inside the body 110. In addition, the wire 120 is connected to a switch 150 and the joint portion 200 described later and controls the joint portion 200 by a manipulation of the switch 150. Detailed descriptions thereof will be described with the joint portion 200.

In addition, the rod portion 100 further includes a guide groove 130, a guide rail 140, a switch 150, and a washing tube 160.

The guide groove 130 is formed at one side of an outer circumferential surface of the body 110, and the switch 150 described later is located at the guide groove 130.

The guide rail 140 is formed inside the guide groove 130, and the guide rail 140 includes a bar-shaped rail connecting between a protrusion and a protrusion protruding from both inner ends of the guide groove 130.

The switch 150 is coupled with the guide rail 140 and connected to the wire 120. In addition, the switch 150 is located in the guide groove 130, and when the switch moves in one direction along the guide rail 140, the joint portion 200 described later may link-rotate in one direction.

The washing tube 160 is formed on the other side of the outer circumferential surface of the body 110. Moreover, the washing tube 160 communicates with an inside of the washing tube body 110. In addition, the washing tube 160 may have an advantage of removing foreign substances formed in an interior of the body 100 by injecting a washing liquid to the washing tube 130 even in a state where the rod portion 100 is not disassembled.

In the fine surgical instrument capable of performing a joint movement and a rotational movement according to the embodiment of the present disclosure, the joint portion 200 is formed on one side of the rod portion 100 and is rotated in one direction from one side of the rod portion 100. Here, the expression "rotated in one direction" indicates bending in one direction. It is preferable that the joint portion 200 is formed of a plurality of link joints 210. For example, the plurality of link joints 210 may be provided with a plurality of spherical members, and the spherical members may be disposed in plural at regular intervals. In addition, the link joint may include a joint link corresponding to the spherical member. Moreover, the spherical member may include a spherical insertion hole through which the above-described wire 120 can pass corresponding to the position of the insertion hole so that the wire 120 can pass through the spherical member. In addition, it is preferably that the through hole is formed to have a diameter corresponding to a diameter of the shaft 300 so that the shaft 300 described later can pass through.

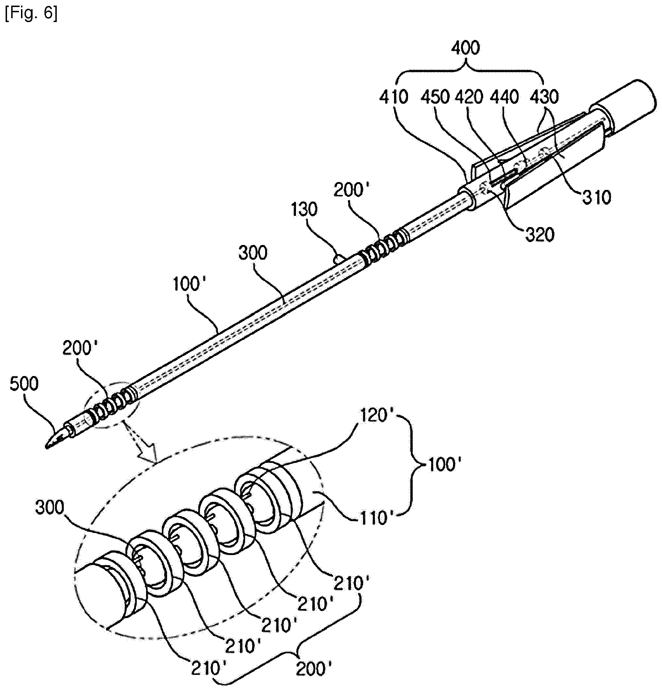

As shown in FIGS. 5 and 6, the joint portion 200 can be link-rotated in one direction by the plurality of link joints 210. In other words, in the joint portion 200, a rotation angle of the joint portion 200 can be adjusted by a manipulation of the switch 150. More specifically, one side of the wire 120 is connected to the plurality of link joints 210 and the other side thereof is connected to the switch 150. If the switch 150 is moved in one direction, as the wire 120 is pulled, the joint portion formed by connecting the plurality of link joints 210 to each other is rotated in the direction in which the wire 120 is pulled.

Meanwhile, if the switch 150 is moved in the other direction, the pulled wire 120 is released, and the joint portion 200 is returned to an original position thereof by an elastic force of the shaft 300.

In this way, the joint portion 200 is manufactured to be rotatable in one direction, and thus, the distal end portion 500 described later is also rotated in the one direction together.

In the fine surgical instrument capable of performing a joint movement and a rotational movement according to the embodiment of the present disclosure, the shaft 300 is inserted into the rod portion 100, and thus, the shaft 300 can linearly reciprocate inside the rod portion 100 so as to approach the treatment site of the patient during the surgery. In this end, the shaft 300 includes a rod portion 310, a first stopper 320 and a second stopper 330.

The rod portion 310 extends in the longitudinal direction and is formed longer than the rod portion 100, and thus, one side of the rod portion 310 is inserted into the rod portion 100. In addition, the other side of the rod portion 310 is coupled with the handle portion 400 described later.

Here, the rod portion 310 may be made of an elastic material, may pass through the above-described joint portion 200, and can be coupled with the distal end portion 500 described later. Accordingly, the shaft 300 may be rotated in response to the link rotation of the joint portion 200. As the rod part 310 linearly reciprocates inside the rod portion 100, the shaft 300 does not come into direct contact with the treatment side of the patient, and thus, is possible to minimize discomfort of the patient.

The first stopper 320 is formed at a distal end portion of the rod portion 310. In addition, the first stopper 320 is formed to protrude from an outer circumferential surface of the rod portion 310 and functions to limit a movement distance of the connection portion 440 described later.

The second stopper 330 is located to be separated from the first stopper 320. The second stopper 330 has the same shape as that of the first stopper 320 and is coupled with a spring 450 to be described later. Moreover, the second stopper 330 serves to prevent the spring 450 is separated from the rod portion 310.

In the fine surgical instrument capable of performing a joint movement and a rotational movement according to the embodiment of the present disclosure, the handle portion 400 may be coupled with the one end of the shaft 300 to rotate the shaft 300. Moreover, the handle portion serves to control a manipulation of the distal end portion 500. To this end, the handle portion 400 includes a body portion 410, a sliding groove 420, a grip portion 430, and a connection portion 440.

In addition, the handle portion 400 may further include the spring 450. Here, the spring 450 is disposed inside the body portion 410 and encloses the shaft 300.

Referring to FIGS. 1 and 5, the body portion 410 has a tubular shape, is formed to extend in the longitudinal direction, and is coupled with the one end of the shaft 300.

The sliding grooves 420 are formed on both sides of the body portion 410.

The grip portions 430 are formed on both sides of the body portion 410, one side of each of the grip portions 430 is coupled with the body portion 410, and the other side thereof is formed to be separated from the sliding groove 420. In addition, the grip portions 430 are formed in a V shape from a rear end of the body portion 410 in a plan view.

The connection portion 440 is connected to an inside of the grip portion 430 and is inserted into the sliding groove 420. In other words, the connection portion 440 includes a connection body 441 which is coupled with the rod portion 310 and is formed in a cylindrical shape, and a connection member 442 which extends from one side of an outer circumferential surface of the connection body 441 and is connected to an inside of the grip portion 430. The connection body 441 slides in one direction along the rod portion 310 when the grip portions 430 are gripped.

Meanwhile, when the gripped grip portion 430 is released, the connection portion 440 can be returned to an original position thereof by the spring 450 while the connection portion 440 reciprocates in the shaft 300.

The distal end portion 500 is coupled with the other end of the shaft 300 and is connected to the handle portion 400 to be operated according to the manipulation of the handle portion 400.

More specifically, the distal end portion 500 may have a forceps ('grab') shape capable of capturing an object of the treatment site or the surgery site. Moreover, the distal end portion 550 may be connected to a rear end of the forceps and may include an 'X' shaped bellows link (not shown) to stretch the forceps. In this case, a distal end of the bellows link can be coupled with the other end of the shaft 300. A plurality of wires are provided at the other end of the shaft 300, and the plurality of wires are coupled with the connection body 441.

Meanwhile, in order to prevent the other end of the shaft 300 from protruding from the rod portion 100, a shaft stopping jaw may be formed inside one end of the body 110.

Accordingly, when the distal end portion 500 grips the grip 430, the connection body 441 moves in one direction through the sliding groove 420. In this case, the connection body 441 linearly moves in one direction in the rod portion 310. Moreover, the plurality of wires connected to the connection body 441 are stretched which pulling the bellows link. In this case, the distal end portion 500 coupled with the bellows link can pick up the treatment site or the surgical site.

In addition, when the grip part 430 is released, the plurality of wires connected to the connection body 441 releases the stretched bellows link while the connection body 441 is returned to the original position thereof by the spring 450.

Meanwhile, when the handle portion 400 is rotated in one direction, the distal end portion 500 can rotate about the shaft 300.

Accordingly, the fine surgical instrument capable of performing a joint movement and a rotational movement according to the embodiment of the present disclosure has an advantage that the joint portion 200 and the handle portion 400 are manipulated such that the instrument can perform a link rotation in one direction and a rotational movement about the shaft 300.

FIGS. 6 to 9 are conceptual views showing a state where a fine surgical instrument capable of performing a joint movement and a rotational movement according to another embodiment of the present disclosure is operated.

In the fine surgical instrument capable of performing a joint movement and a rotational movement according to another embodiment of the present disclosure, the above descriptions are referred to the same components as the components of the above-described embodiment, a rod portion 100' and a joint portion 200' which are modified components will be described in more detail.

Therefore, in FIGS. 6 to 8, similar reference numerals will be given to the same or corresponding components as those of the embodiment, and detailed descriptions of these components will be omitted below.

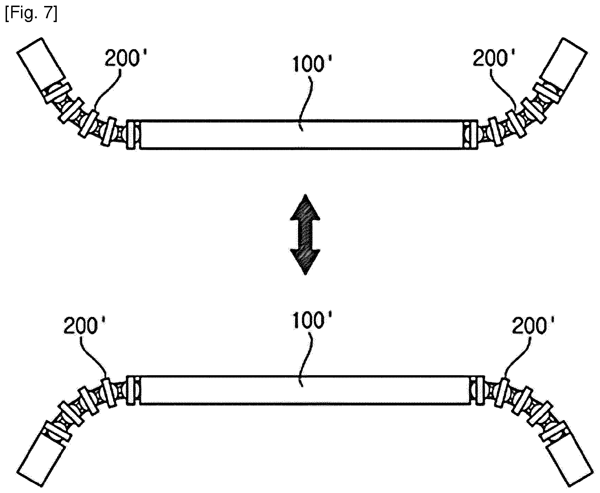

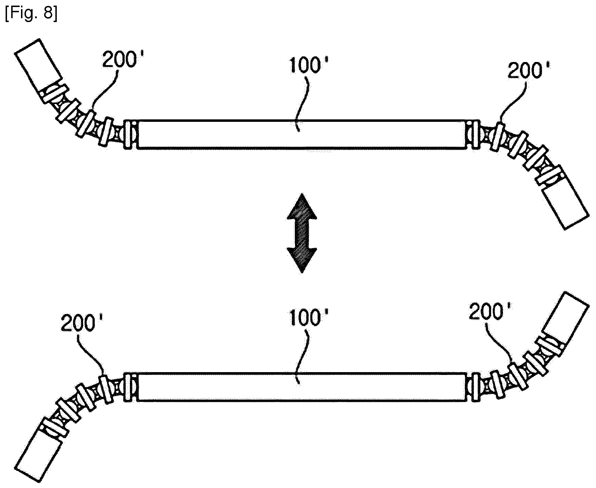

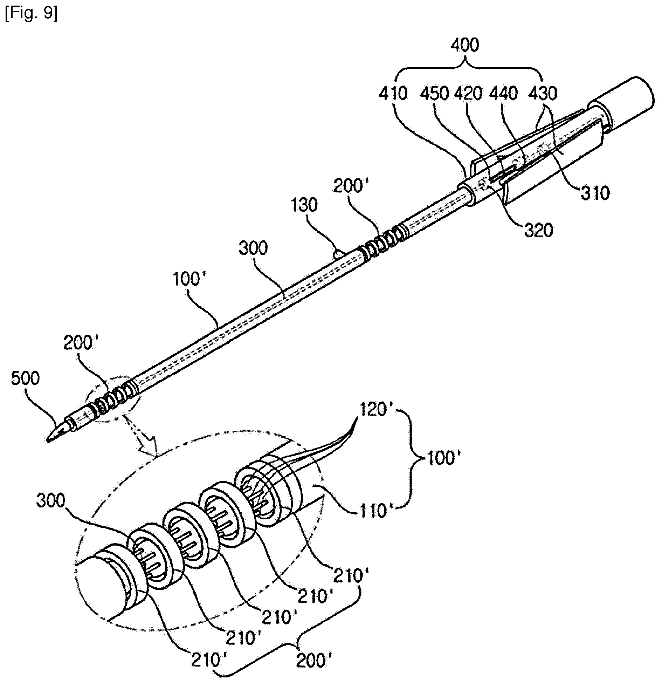

FIG. 6 is a perspective view schematically showing an appearance of the fine surgical instrument capable of performing a joint movement and a rotational movement according to another embodiment of the present disclosure, FIG. 7 is a conceptual view showing a shape in which a pair of joint portions is link-rotated in the same direction, FIG. 8 is a conceptual view showing a shape in which the pair of joint portions is link-rotated in different directions, and FIG. 9 is a perspective view schematically showing a shape in which a plurality of link joints and a plurality of wires are coupled.

Referring to FIG. 6, the fine surgical instrument capable of performing a joint movement and a rotational movement according to another embodiment of the present disclosure may further include a wire 120' which is formed inside the rod portion 100' and the pair of joint portions 200' which is formed on both ends of the rod portion 100'.

The wire 120' is formed inside the rod portion 100' and may be connected to the pair of joint portions 200'.

The pair of joint portions 200' is formed on both ends of the rod portion 100', and is preferably formed of a plurality of link joints 210'.

For example, the plurality of link joints 210' may have a plurality of spherical members and may include joint links corresponding to the spherical members. In addition, each of the spherical members may include an insertion hole through which the above-described wire can pass.

As shown in FIG. 7 and FIG. 8, the pair of joint portions 200' may have an advantage that joint portions 200' can be link-rotated in the same direction or in different directions. In this way, the pair of joint portions 200' has an advantage of facilitating surgery of the user.

As shown in FIG. 9, the rod portion 100' has a tubular shape extending in a longitudinal direction, and a plurality of the wires 120' may be formed along the longitudinal direction inside the rod portion 100'. Moreover, a plurality of wires may be coupled with the plurality of link joints 210'.

For example, the plurality of link joints 210' may have a plurality of spherical members and may include joint links corresponding to the spherical members. In addition, the spherical members may include a plurality of insertion holes through which the plurality of wires can pass.

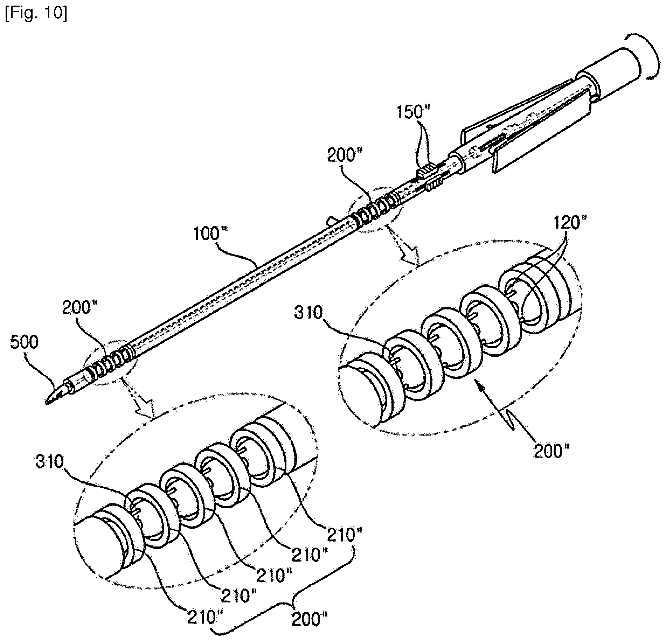

FIGS. 10 to 12 are conceptual views showing a state where a fine surgical instrument capable of performing a joint movement and a rotational movement according to still another embodiment of the present disclosure is operated.

In the fine surgical instrument capable of performing a joint movement and a rotational movement according to still another embodiment of the present disclosure, the above descriptions are referred to the same components as the components of the above-described embodiment, a pair of joint portions 200" which is a modified component will be described in more detail.

Therefore, in FIGS. 10 to 12, similar reference numerals will be given to the same or corresponding components as those of the embodiment, and detailed descriptions of these components will be omitted below.

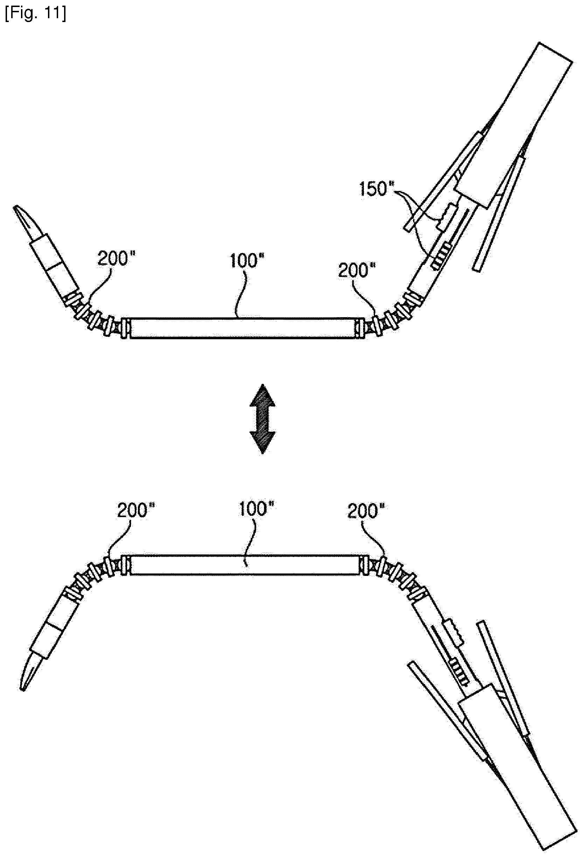

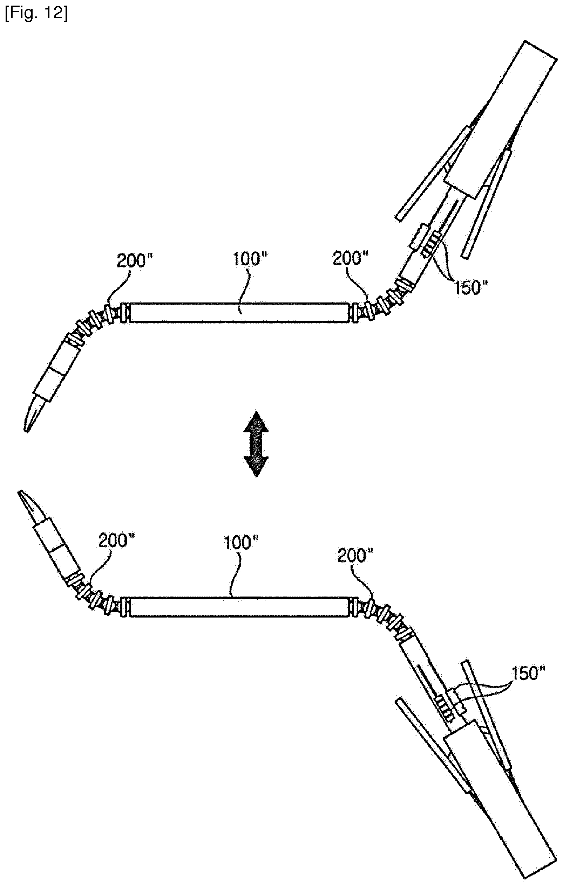

FIG. 10 is a perspective view schematically showing an appearance of the fine surgical instrument capable of performing a joint movement and a rotational movement according to still another embodiment of the present disclosure, FIG. 11 is a conceptual view showing a shape in which a pair of joint portions of FIG. 10 is link-rotated in the same direction, and FIG. 12 is a conceptual view showing a shape in which the pair of joint portions of FIG. 10 is link-rotated in different directions.

Referring to FIG. 10, the fine surgical instrument capable of performing a joint movement and a rotational movement according to still another embodiment of the present disclosure may further include the pair of joint portions 200" which is formed on both ends of a rod portion 100".

The rod portion 100" has a tubular shape extending in a longitudinal direction, and a plurality of the wires may be formed along the longitudinal direction inside the rod portion 100". Moreover, the rod portion 100" include a multi-switch 150" connected to a plurality of wires 120". Meanwhile, the plurality of wires may be coupled with the pair of joint portions 200".

It is preferable that the pair of joint portions 200" is formed on both ends of the rod portions 100" and includes a plurality of link joints 210".

As shown in FIGS. 10 and 11, the pair of joint portions 200" may have an advantage that the joint portions 200" are link-rotated in the same direction or different directions by moving the multi-switch 150" in one direction or the other direction.

In this way, the pair of joint portions 200" has an advantage of facilitating surgery of the user. That is, when the endoscope and the surgery instrument together enter a narrow space while surgery is performed on a base of a brain through a nasal cavity, the pair of joint portions 200" is link-rotated in respective directions, and thus, it is possible to minimize interference between the endoscope and the surgery instrument. Accordingly, it is possible to freely perform the surgery on the treatment site of the patient.

The above-described description of the present disclosure is for illustrative purposes, and it will be understood by those skilled in the art that the present disclosure can be easily modified into other specific forms without changing a technical spirit or essential characteristics of the present disclosure. Therefore, it should be understood that the above-described embodiments are exemplary in all respects and not restrictive. For example, each component described as a single type may be implemented in a distributed manner, and similarly, components described as distributed may be implemented in a coupled form.

The scope of the present disclosure is indicated by the following claims, and all changes or modifications derived from the meaning and scope of the claims and their equivalent concepts should be interpreted as being included in the scope of the present disclosure.

According the fine surgical instrument capable of performing a joint movement and a rotational movement of the present disclosure, the pair of joint portions is disposed on both sides of the rod portion so as to be link-rotated in each direction, and thus, the fine surgical instrument can be finely manipulated without colliding with the endoscope in a narrow space during the endoscope surgery.

In addition, according the fine surgical instrument capable of performing a joint movement and a rotational movement of the present disclosure, when the handle portion rotates in one direction, the distal end portion is also rotated in the one direction together, and thus, it is possible to easily control the instrument.

Moreover, according the fine surgical instrument capable of performing a joint movement and a rotational movement of the present disclosure, the number of drive units and the number of components of the surgical instrument are reduced, and thus, it is possible to secure a price competitiveness of the surgical instrument.

## Claims

1. A fine surgical instrument capable of performing a joint movement and a rotational movement, the fine surgical instrument comprising:
a rod portion (100, 100', 100") which is formed in a longitudinal direction to enter a surgery site located in a narrow space;
a joint portion (200, 200', 200") which is formed on one side of the rod portion (100, 100', 100") and which is configured to rotate in one direction from the one side of the rod portion (100, 100', 100");
a shaft (300) which is inserted into the rod portion (100, 100', 100") and which is provided with a rod (300) including a rod portion (310);
a handle portion (400) which is coupled with the other side of the rod portion (100, 100', 100"); and
a distal end portion (500) which is coupled with one end of the shaft (300) and is connected to the handle portion (400),
wherein the distal end portion (500) is operated along a manipulation of the handle portion (400),
wherein the handle portion (400) includes a tubular body portion (410), sliding grooves (420) which are formed on both sides of the body portion (410), a grip portion (430) of which one side is coupled with the body portion (410) and the other side is formed to be separated from the sliding grooves (420), a connection portion (440) which is connected to an inside of the grip portion (430) and is inserted into the sliding grooves (420), and a spring (450) which is inserted into the body portion (410),
wherein the connection portion (440) includes a connection body (441) which is coupled with the rod portion (310) and is formed in a cylindrical shape, and a connection member (442) which extends from one side of an outer circumferential surface of the connection body (441) and is connected to the inside of the grip portion (430), and
wherein the connection body (441) slides in one direction along the rod portion (310) when the grip portion (430) is gripped.

2. The fine surgical instrument of claim 1, wherein the rod portion (100, 100', 100") is a tubular body extending in the longitudinal direction and includes a wire (120, 120', 120") formed inside the body.

3. The fine surgical instrument of claim 2, wherein the rod portion (100, 100', 100") further includes a guide groove (130) which is formed on one side of an outer peripheral surface of the body, a guide rail (140) which is formed inside the guide groove (130), a switch (150) which is coupled with the guide rail (140) and is connected to the wire (120, 120', 120"), and a washing tube (160) which is formed on the other side of the outer peripheral surface of the body.

4. The fine surgical instrument of claim 1, wherein the joint portion (200, 200', 200") includes a plurality of link joints (210, 210', 210").

5. The fine surgical instrument of claim 3, wherein when the switch (150) moves in one direction, the joint portion (200, 200', 200") is link-rotated in one direction.

6. The fine surgical instrument of claim 4, wherein the shaft (300) includes a first stopper (320) which is formed at a distal end portion of the rod portion (310), and a second stopper (330) which is located to be separated from the first stopper (320).

7. The fine surgical instrument of claim 6, wherein the rod portion (310) is formed of an elastic material.

8. The fine surgical instrument of claim 1, wherein the distal end portion (500) rotates about the shaft (300) when the handle portion (400) rotates in one direction.

9. The fine surgical instrument of claim 1, further comprising:
a wire (120, 120', 120") which is formed inside the rod portion (100, 100', 100"); and
a pair of joint portions (200, 200', 200") which is formed on both ends of the rod portion (100, 100', 100").

10. The fine surgical instrument of claim 9, wherein a plurality of the wires (120, 120, 120") may be formed along the longitudinal direction inside the rod portion (100, 100', 100").

11. The fine surgical instrument of claim 9, wherein the pair of joint portions (200, 200', 200") includes a plurality of link joints (210, 210', 210").

12. The fine surgical instrument of claim 11, wherein the plurality of link joints (210, 210', 210") are coupled with the plurality of wires (120, 120', 120").

## Patentansprüche

1. Feinchirurgisches Instrument, das in der Lage ist, eine Gelenkbewegung und eine Drehbewegung auszuführen, wobei das feinchirurgische Instrument Folgendes umfasst:
einen Stangenabschnitt (100, 100', 100"), der in Längsrichtung ausgebildet ist, um in eine Operationsstelle einzudringen, die sich in einem engen Raum befindet;
einen Gelenkabschnitt (200, 200', 200"), der auf einer Seite des Stangenabschnitts (100, 100', 100") ausgebildet ist und der dazu konfiguriert ist, sich von der einen Seite des Stangenabschnitts (100, 100', 100") in eine Richtung zu drehen;
einen Schaft (300), der in den Stangenabschnitt (100, 100', 100") eingesetzt ist und der mit einer Stange (300) versehen ist, die einen Stangenabschnitt (310) beinhaltet;
einen Griffabschnitt (400), der mit der anderen Seite des Stangenabschnitts (100, 100', 100") gekoppelt ist; und
einen distalen Endabschnitt (500), der mit einem Ende des Schafts (300) gekoppelt und mit dem Griffabschnitt (400) verbunden ist,
wobei der distale Endabschnitt (500) entsprechend einer Betätigung des Griffabschnitts (400) betrieben wird,
wobei der Griffabschnitt (400) einen röhrenförmigen Körperabschnitt (410), Gleitnuten (420), die auf beiden Seiten des Körperabschnitts (410) ausgebildet sind, einen Griffabschnitt (430), dessen eine Seite mit dem Körperabschnitt (410) gekoppelt ist und die andere Seite so ausgebildet ist, dass sie von den Gleitnuten (420) getrennt ist, einen Verbindungsabschnitt (440), der mit einer Innenseite des Griffabschnitts (430) verbunden ist und in die Gleitnuten (420) eingesetzt ist, und eine Feder (450), die in den Körperabschnitt (410) eingesetzt ist, beinhaltet,
wobei der Verbindungsabschnitt (440) einen Verbindungskörper (441), der mit dem Stangenabschnitt (310) gekoppelt ist und in einer zylindrischen Form ausgebildet ist, und ein Verbindungselement (442), das sich von einer Seite einer äußeren Umfangsfläche des Verbindungskörper (441) erstreckt und mit der Innenseite des Griffabschnitts (430) verbunden ist, beinhaltet und
wobei der Verbindungskörper (441) in einer Richtung entlang des Stangenabschnitts (310) gleitet, wenn der Griffabschnitt (430) gegriffen wird.

2. Feinchirurgisches Instrument nach Anspruch 1, wobei der Stababschnitt (100, 100', 100") ein röhrenförmiger Körper ist, der sich in Längsrichtung erstreckt und einen im Inneren des Körpers ausgebildeten Draht (120, 120', 120") beinhaltet.

3. Feinchirurgisches Instrument nach Anspruch 2, wobei der Stababschnitt (100, 100', 100") ferner eine Führungsnut (130), die auf einer Seite einer Außenumfangsfläche des Körpers ausgebildet ist, eine Führungsschiene (140), der innerhalb der Führungsnut (130) ausgebildet ist, einen Schalter (150), der mit der Führungsschiene (140) gekoppelt und mit dem Draht (120, 120', 120") verbunden ist, und ein Waschrohr (160), das auf der anderen Seite der Außenumfangsfläche des Körpers gebildet ist.

4. Feinchirurgisches Instrument nach Anspruch 1, wobei der Gelenkabschnitt (200, 200', 200") eine Vielzahl von Gliedergelenken (210, 210', 210") beinhaltet.

5. Feinchirurgisches Instrument nach Anspruch 3, wobei, wenn sich der Schalter (150) in eine Richtung bewegt, der Gelenkabschnitt (200, 200', 200") gliedrig in eine Richtung gedreht wird.

6. Feinchirurgisches Instrument nach Anspruch 4, wobei der Schaft (300) einen ersten Anschlag (320), der an einem distalen Endabschnitt des Stangenabschnitts (310) ausgebildet ist, und einen zweiten Anschlag (330), der so angeordnet ist, dass er von dem ersten Anschlag (320) getrennt werden kann, beinhaltet.

7. Feinchirurgisches Instrument nach Anspruch 6, wobei der Stababschnitt (310) aus einem elastischen Material ausgebildet ist.

8. Feinchirurgisches Instrument nach Anspruch 1, wobei sich der distale Endabschnitt (500) um den Schaft (300) dreht, wenn sich der Griffabschnitt (400) in eine Richtung dreht.

9. Feinchirurgisches Instrument nach Anspruch 1, ferner umfassend:
einen Draht (120, 120', 120"), der innerhalb des Stababschnitts (100, 100', 100") ausgebildet ist; und
ein Paar Gelenkabschnitte (200, 200', 200"), das an beiden Enden des Stangenabschnitts (100, 100', 100") ausgebildet ist.

10. Feinchirurgisches Instrument nach Anspruch 9, wobei eine Vielzahl von Drähten (120, 120, 120") entlang der Längsrichtung innerhalb des Stababschnitts (100, 100', 100") ausgebildet sein können.

11. Feinchirurgisches Instrument nach Anspruch 9, wobei das Paar Gelenkabschnitte (200, 200', 200") eine Vielzahl von Gliederverbindungen (210, 210', 210") beinhaltet.

12. Feinchirurgisches Instrument nach Anspruch 11, wobei die Vielzahl von Gliederverbindungen (210, 210', 210") mit der Vielzahl von Drähten (120, 120', 120") gekoppelt ist.

## Revendications

1. Instrument chirurgical fin capable d'effectuer un mouvement articulaire et un mouvement de rotation, l'instrument chirurgical fin comprenant :
une partie de tige (100, 100', 100") qui est formée dans une direction longitudinale pour pénétrer dans un site chirurgical situé dans un espace étroit ;
une partie articulaire (200, 200', 200") qui est formée sur un côté de la partie de tige (100, 100', 100") et qui est configurée pour tourner dans une direction à partir de ce côté de la partie de tige (100, 100', 100") ;
un axe (300) qui est inséré dans la partie de tige (100, 100', 100") et qui est pourvu d'une tige (300) comprenant une partie de tige (310);
une partie de poignée (400) qui est couplée à l'autre côté de la partie de tige (100, 100', 100") ; et
une partie d'extrémité distale (500) qui est couplée à une extrémité de l'axe (300) et est connectée à la partie de poignée (400),
dans lequel la partie d'extrémité distale (500) est actionnée lors d'une manipulation de la partie de poignée (400),
dans lequel la partie de poignée (400) comprend une partie de corps tubulaire (410), des rainures coulissantes (420) qui sont formées des deux côtés de la partie de corps (410), une partie de préhension (430) dont un côté est couplé à la partie de corps (410) et l'autre côté est formé pour être séparé des rainures coulissantes (420), une partie de connexion (440) qui est connectée à un intérieur de la partie de préhension (430) et est insérée dans les rainures coulissantes (420), et un ressort (450) qui est inséré dans la partie de corps (410),
dans lequel la partie de connexion (440) comprend un corps de connexion (441) qui est couplé à la partie de tige (310) et est formé dans une forme cylindrique, et un élément de connexion (442) qui s'étend depuis un côté d'une surface circonférentielle externe du corps de connexion (441) et est connecté à l'intérieur de la partie de préhension (430), et
dans lequel le corps de connexion (441) coulisse dans une direction le long de la partie de tige (310) lorsque la partie de préhension (430) est saisie.

2. Instrument chirurgical fin selon la revendication 1, dans lequel la partie de tige (100, 100', 100") est un corps tubulaire s'étendant dans la direction longitudinale et comprend un fil (120, 120', 120") formé à l'intérieur du corps.

3. Instrument chirurgical fin selon la revendication 2, dans lequel la partie de tige (100, 100', 100") comprend en outre une rainure de guidage (130) qui est formée sur un côté d'une surface périphérique externe du corps, un rail de guidage (140) qui est formé à l'intérieur de la rainure de guidage (130), un commutateur (150) qui est couplé au rail de guidage (140) et est connecté au fil (120, 120', 120"), et un tube de lavage (160) qui est formé sur l'autre côté de la surface périphérique externe du corps.

4. Instrument chirurgical fin selon la revendication 1, dans lequel la partie articulaire (200, 200', 200") comprend une pluralité d'articulations de liaison (210, 210', 210").

5. Instrument chirurgical fin selon la revendication 3, dans lequel lorsque le commutateur (150) se déplace dans une direction, la partie articulaire (200, 200', 200") est entraînée en rotation dans cette direction.

6. Instrument chirurgical fin selon la revendication 4, dans lequel l'axe (300) comprend une première butée (320) qui est formée au niveau d'une partie d'extrémité distale de la partie de tige (310), et une seconde butée (330) qui est située pour être séparée de la première butée (320).

7. Instrument chirurgical fin selon la revendication 6, dans lequel la partie de tige (310) est formée d'un matériau élastique.

8. Instrument chirurgical fin selon la revendication 1, dans lequel la partie d'extrémité distale (500) tourne autour de l'axe (300) lorsque la partie de poignée (400) tourne dans une direction.

9. Instrument chirurgical fin selon la revendication 1, comprenant en outre :
un fil (120, 120', 120") qui est formé à l'intérieur de la partie de tige (100, 100', 100") ; et
une paire de parties articulaires (200, 200', 200") qui est formée sur les deux extrémités de la partie de tige (100, 100', 100").

10. Instrument chirurgical fin selon la revendication 9, dans lequel une pluralité de fils (120, 120, 120") peuvent être formés le long de la direction longitudinale à l'intérieur de la partie de tige (100, 100', 100").

11. Instrument chirurgical fin selon la revendication 9, dans lequel la paire de parties articulaires (200, 200', 200") comprend une pluralité d'articulations de liaison (210, 210', 210").

12. Instrument chirurgical fin selon la revendication 11, dans lequel la pluralité d'articulations de liaison (210, 210', 210") sont couplées à la pluralité de fils (120, 120', 120").
